# EUROPÄISCHE PATENTANMELDUNG

(11) **EP 3 284 733 A1**
(43) Veröffentlichungstag der Anmeldung: **21.02.2018**
(21) Anmeldenummer: 16184562.3
(22) Anmeldetag: 17.08.2016
(51) Int. Cl.: C07C 29/151, C07C 31/04, C10K 1/12, C01B 3/02

(54) **VERFAHREN ZUR SYNTHESE VON METHANOL**

(71) Anmelder: Wagner, Ulrich, 06406 Bernburg (DE); Balthasar, Wolff, 40833 Ratingen (DE); Müller, Dierk, 61184 Karben (DE)
(72) Erfinder: Wagner, Ulrich, 06406 Bernburg (DE); Balthasar, Wolff, 40833 Ratingen (DE); Müller, Dierk, 61184 Karben (DE)
(74) Vertreter: Patentanwälte Bauer Vorberg Kayser

(57) **Zusammenfassung**

Die Erfindung betrifft ein Verfahren zur Synthese von Methanol, wobei aus einem Gasstrom (1) mit Wasserstoff und Kohlenstoffdioxid das Kohlenstoffdioxid zumindest teilweise durch ein Waschmedium (2) ausgewaschen wird und wobei nach dem Auswaschen der Gasstrom (1) einer Reaktoranordnung (3) zur katalytischen und zumindest teilweisen Umsetzung von Wasserstoff und Kohlenstoffoxiden in ein Gasgemisch mit Wasser und Methanol zugeführt wird. Das Verfahren ist dadurch gekennzeichnet, dass das Waschmedium (2) eine wässrige Lösung (2a) von Ammoniak zum Auswaschen des Kohlenstoffdioxids umfasst. Ebenso betrifft die Erfindung eine entsprechende Anlage zur Synthese von Methanol.

## Beschreibung

Die Erfindung betrifft ein Verfahren zur Synthese von Methanol gemäß dem Oberbegriff von Anspruch 1 sowie eine Anlage zur Synthese von Methanol gemäß dem Oberbegriff von Anspruch 15.

Methanol wird regelmäßig in industriellen Anlagen hergestellt, in welchen fossile Energieträger wie etwa Kohle, Erdgas oder spezielle Gase mit einzelnen Alkanen aus dem Erdgas zunächst in ein Synthesegas aus Wasserstoff und Kohlenstoffoxiden umgewandelt werden. Anschließend kann eine katalytische Umwandlung des Synthesegases in ein Rohmethanol umfassend Methanol und Wasser in einem entsprechenden Reaktor stattfinden.

Je nachdem welcher oder welche fossile Energieträger den Ausgangsstoff für die Methanolsynthese bilden und ebenfalls je nach angewandter Art der Umwandlung der fossilen Energieträger in das Synthesegas kann das Synthesegas unterschiedliche molare Anteile von Wasserstoff, Kohlenstoffmonoxid und Kohlenstoffdioxid aufweisen. Bevorzugt wird dabei für die Methanolsynthese ein durch S gemäß der Formel S = (n(H₂) - n(CO₂)) / (n(CO) + n(CO₂)) mit n in [mol] gegebenes molares Verhältnis von im Wesentlichen 2,1 angestrebt. Dieses molare Verhältnis wird auch als Stöchiometriezahl bezeichnet.

Es kann allerdings sein, dass das Synthesegas gegenüber dieser angestrebten Stöchiometriezahl eine abweichende Zusammensetzung aufweist, wobei regelmäßig ein Überschuss an Kohlenstoffdioxid vorliegen kann. Das Ausmaß dieser Abweichung kann insbesondere bei Erdgas als Energieträger, welches je nach Quelle deutlich unterschiedliche Anteile von Methan und höheren Kohlenwasserstoffen aufweisen kann, ebenso deutlich variieren.

Regelmäßig wird in solchen Anlagen zur Synthese von Methanol das unreagierte Gas aus dem Reaktor - welches neben dem ggf. überschüssigen Kohlenstoffdioxid auch unreagierten Wasserstoff und unreagiertes Kohlenstoffmonoxid umfasst - als Recyclegasstrom neben dem frischen Synthesegas - auch als Frischgas bezeichnet - dem Reaktor zugeführt. Ein bereits im frischen Synthesegas vorhandener Überschuss an Kohlenstoffdioxid wird durch die Rückführung des ebenfalls mit überschüssigem Kohlenstoffdioxid behafteten Restgases verschärft.

Hinzu kommt, dass Kohlenstoffdioxid für die Methanolsynthese eine geringere Reaktivität aufweist als Kohlenstoffmonoxid. Daher muss bei einem höheren Anteil an Kohlenstoffdioxid auch eine größere Menge an unreagiertem Gas insgesamt im Kreislauf gefahren werden. Dies erfordert eine entsprechend große Dimensionierung der hierzu erforderlichen Kompressoren, was wiederum mit einer hohen Leistungsaufnahme einhergeht. Es versteht sich, dass dies die Wirtschaftlichkeit der Methanolsynthese beeinträchtigt.

Um diese Situation zu verbessern, schlägt die aus dem Stand der Technik bekannte EP 0 067 491 B1, von welcher die vorliegende Erfindung ausgeht, vor, das Kohlenstoffdioxid in dem gewünschten Ausmaß aus dem Synthesegasstrom auszuwaschen. Speziell soll dies durch einen Nassreinigungsvorgang unter Verwendung von noch nicht expandiertem Rohmethanol aus dem Reaktor für die Methanolsynthese erfolgen. Als vorteilhaft wird dabei beschrieben, dass das Waschmedium durch den Syntheseprozess selbst bereitgestellt wird und dass eine gesonderte Regeneration für das Waschmedium entfallen kann, da das Kohlenstoffdioxid einfach durch eine Expansion des Rohmethanols freigesetzt werden soll. Anschließend kann das für die Wäsche verwendete Rohmethanol der Reinigungsstufe zugeführt werden, welche ohnehin für das synthetisierte Methanol vorgesehen ist. Da das Rohmethanol also auch nicht wieder komprimiert zu werden braucht, wird dieser Ansatz als energetisch sparsam angesehen.

Nachteilig hieran ist allerdings, dass das bloße Freisetzen des ausgewaschenen Kohlenstoffdioxids als Treibhausgas umweltschädlich ist. Ebenso ist der obige Ansatz hinsichtlich des Reinigungsgrads an ausgewaschenem Kohlenstoffdioxid beschränkt.

Ausgehend von diesem Stand der Technik besteht die Aufgabe der Erfindung folglich darin, das aus dem Stand der Technik bekannte Verfahren und die aus dem Stand der Technik bekannte Anlage zur Synthese von Methanol hinsichtlich ihrer Wirtschaftlichkeit und ihrer Wirkung auf die Umwelt zu verbessern.

Bezogen auf ein Verfahren zur Synthese von Methanol gemäß dem Oberbegriff von Anspruch 1 wird diese Aufgabe durch die Merkmale des kennzeichnenden Teils von Anspruch 1 gelöst. Bezogen auf eine Anlage zur Synthese von Methanol gemäß dem Oberbegriff von Anspruch 15 wird die Aufgabe durch die Merkmale des kennzeichnenden Teils von Anspruch 15 gelöst.

Der Erfindung liegt die Erkenntnis zugrunde, dass für die Kohlenstoffdioxid-Wäsche aus einem Gasstrom, welcher zumindest teilweise den Ausgangsstoff für die Synthese von Methanol bereitstellt, eine Ammoniaklösung verwendet werden kann. Es handelt sich bei diesem Gasstrom also im Prinzip um ein Gasgemisch mit Wasserstoff und Kohlenstoffoxiden, welches ganz oder teilweise und allein oder nach Zusammenführung mit anderen Gasen einem Reaktor für die Methanolsynthese zugeführt wird. Insbesondere kann es sich um ein frisches - also aus der Synthese kommendes und noch gar nicht einem Reaktor zugeführtes - Gasgemisch mit Wasserstoff und Kohlenstoffoxiden, welches dann auch als Frischgas bezeichnet wird, um ein als Restgas aus dem Reaktor zurückgeführtes und damit rezykliertes Recyclegas mit Wasserstoff und Kohlenstoffoxiden oder um eine Mischung aus einem solchen Frischgas und einem solchen Recyclegas handeln. Der Gasstrom insgesamt, welcher dem Reaktor oder einer Anordnung von mehreren Reaktoren zur Methanolsynthese zugeführt wird, wird auch als Speisestrom bezeichnet.

Ammoniak ist ein in seiner Herstellung vergleichsweise günstiger Stoff und bietet gleichzeitig einen hohen Reinigungsgrad für die Kohlenstoffdioxidwäsche. Ebenfalls wirtschaftlich günstig kann die Regeneration der Waschlösung gestaltet werden. Die potenziell nachteiligen Verluste von Ammoniak, begründet durch den hohen Dampfdruck von Ammoniak, werden dadurch wirksam begrenzt, dass das zu waschende Gas des Gasstroms selbst mit einem hohen Druck der Kohlenstoffdioxid-Wäsche unterzogen wird. Das führt zusätzlich dazu, dass auch das ausgewaschene Kohlenstoffdioxid in der Regeneration mit einem hohen Druck abgegeben werden kann. Dieser hohe Druck bei der Abgabe erleichtert eine Sequestrierung des Kohlenstoffdioxids, z.B. durch ein Verpressen in eine unterirdische Aufnahme.

Die bevorzugte Ausgestaltung des Unteranspruchs 3 sieht dabei vor, dass ein Recyclegasstrom mit Restgas aus dem Reaktor der vorschlagsgemäßen Kohlenstoffdioxidwäsche unterzogen wird. Das ist vor allem deshalb vorteilhaft, weil dann bereits eine Abkühlung auch des Restgases zum Abscheiden des im Reaktor erzeugten Rohmethanols bereits stattgefunden hat, was für die Wäsche vorteilhaft ist.

Bei einer mehrstufigen Reaktoranordnung kann eine solche Wäsche des Restgases dann entweder an dem Restgas der letzten Stufe vorgenommen werden - was in dem Unteranspruch 4 beschrieben wird - oder an einem Restgas vorgenommen werden, welches zwischen zwei Stufen der mehrstufigen Reaktoranordnung abgetrennt wurde. Dies ist der Gegenstand des Unteranspruchs 5 und hat den Vorteil, dass damit auch die nachfolgenden Reaktorstufen entlastet werden.

Die bevorzugte Ausgestaltung des Unteranspruchs 7 sieht vor, dass es sich bei dem Gasstrom um einen Frischgasstrom handelt, welcher aus einer Reformierung eines kohlenstoffhaltigen Energieträgerstroms gewonnen wird, wobei dann dieser gewonnene Frischgasstrom vor Zuführung zu der Reaktoranordnung der Wäsche unterzogen werden kann. Dies hat den Vorteil, dass in der Reaktoranordnung von vornherein ein Gasgemisch mit einer für die Methanolsynthese günstigeren Stöchiometriezahl vorliegt. Gemäß dem Unteranspruch 8 kann diese Reformierung ein Pre-Reforming und gemäß Unteranspruch 9 eine autotherme Reformierung mit Sauerstoff umfassen.

Die vorteilhaften Ausgestaltungen der Unteransprüche 10 bis 14 betreffen den Waschvorgang im engeren Sinne. So beschreibt der Unteranspruch 10 eine vorteilhafte Zusammensetzung des Waschmediums. Die Unteransprüche 12 und 13 sehen vorteilhafte Ausgestaltungen der Waschstufe zur wirksamen Aufnahme von Kohlenstoffdioxid in der Absorptionsstufe und zur Abgabe unter möglichst hohem Druck in der Regenerationsstufe vor, sodass - wie vom Unteranspruch 14 beschrieben - das abgegebene Kohlenstoffdioxid möglichst günstig in eine unterirdische Lagerstätte verpresst werden kann. Ein solches Verpressen stellt die insbesondere unter ökologischen Gesichtspunkten bevorzugte Behandlung des überschüssigen Kohlenstoffdioxids dar.

Weitere Einzelheiten, Merkmale, Ziele und Vorteile der vorliegenden Erfindung werden nachfolgend anhand der Zeichnung bevorzugter Ausführungsbeispiele erläutert. In der Zeichnung zeigt
- Fig. 1: schematisch das Fließbild einer Anlage zur Ausführung des vorschlagsgemäßen Verfahrens gemäß einem ersten Ausführungsbeispiel und
- Fig. 2: schematisch das Fließbild einer Anlage zur Ausführung des vorschlagsgemäßen Verfahrens gemäß einem zweiten Ausführungsbeispiel.

Das vorschlagsgemäße Verfahren dient der Synthese von Methanol, wobei diese Synthese in an sich aus dem Stand der Technik bekannter Weise erfolgt. Gemäß dem vorschlagsgemäßen Verfahren wird aus einem Gasstrom 1 welcher Wasserstoff und Kohlenstoffdioxid umfasst, das Kohlenstoffdioxid zumindest teilweise durch ein Waschmedium 2 ausgewaschen. Neben Wasserstoff und Kohlenstoffdioxid kann dieser Gasstrom 1 auch weitere Bestandteile, insbesondere Kohlenstoffmonoxid, aufweisen. Zu diesen weiteren Bestandteilen können vor allem auch inerte Gase zählen. Dieser Gasstrom 1 kann dabei prozesstechnisch an ganz unterschiedlichen Stellen der Methanolsynthese anfallen, wie untenstehend noch genauer beschrieben wird.

Vorschlagsgemäß wird der Gasstrom 1 nach dem Auswaschen - also nach dem obigen zumindest teilweisen Auswaschen des Kohlenstoffdioxids - einer Reaktoranordnung 3 zur katalytischen und zumindest teilweisen Umsetzung von Wasserstoff und Kohlenstoffoxiden in ein Gasgemisch mit Wasser und Methanol zugeführt. Unter dem Begriff der Kohlenstoffoxide sind hier und nachfolgend Kohlenstoffmonoxid sowie Kohlenstoffdioxid zu verstehen. Eine solche Methanolsynthese durch katalytische Umsetzung ist an und für sich aus dem Stand der Technik bekannt. In beiden vorliegenden Ausführungsbeispielen umfasst die Reaktoranordnung 3 jeweils einen isothermen Reaktor 3a und einen adiabaten Reaktor 3b, welche an sich ebenfalls aus dem Stand der Technik bekannt sind.

Der umzusetzende Wasserstoff und die umzusetzenden Kohlenstoffoxide können zumindest teilweise von dem Gasstrom 1 bereitgestellt werden. Zwischen dem Auswaschen des Kohlenstoffdioxids und der Zuführung zu der Reaktoranordnung 3 können noch weitere und insbesondere auf den Gasstrom 1 bezogene Verfahrensschritte vorgesehen sein.

Vorschlagsgemäß umfasst das Waschmedium 2 eine wässrige Lösung 2a von Ammoniak zum Auswaschen des Kohlenstoffdioxids. Das Waschmedium 2 kann auch aus dieser Lösung 2a bestehen. Die Lösung 2a kann auch insbesondere aufgelöstes Kohlenstoffdioxid umfassen. Eine solche Lösung 2a kann durch das Auflösen von Ammoniak oder Ammoniumcarbonat in Wasser erlangt werden, wobei Kohlenstoffdioxid insbesondere durch den hier gegenständlichen Waschvorgang in die Lösung 2a gelangt. Insbesondere vor dem Auswaschen des Kohlenstoffdioxids liegt dabei das Kohlenstoffdioxid in dem Waschmedium 2 ungesättigt oder ggf. nur in sehr geringer Konzentration vor. Das Ammoniak und das Kohlenstoffdioxid können auch Ammoniumhydrogencarbonat (NH₄HCO₃) im Wasser bilden.

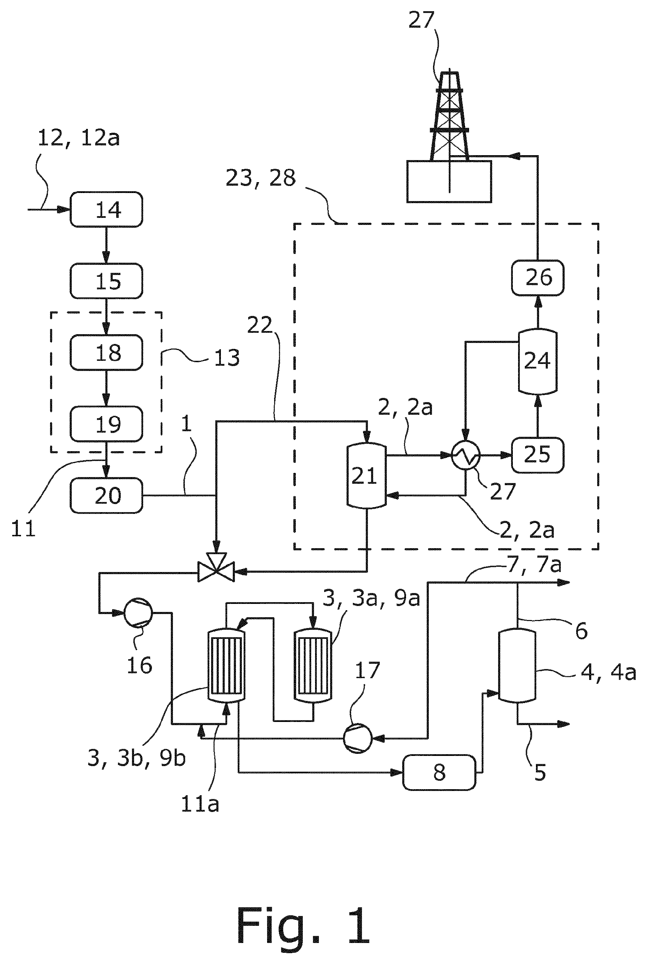

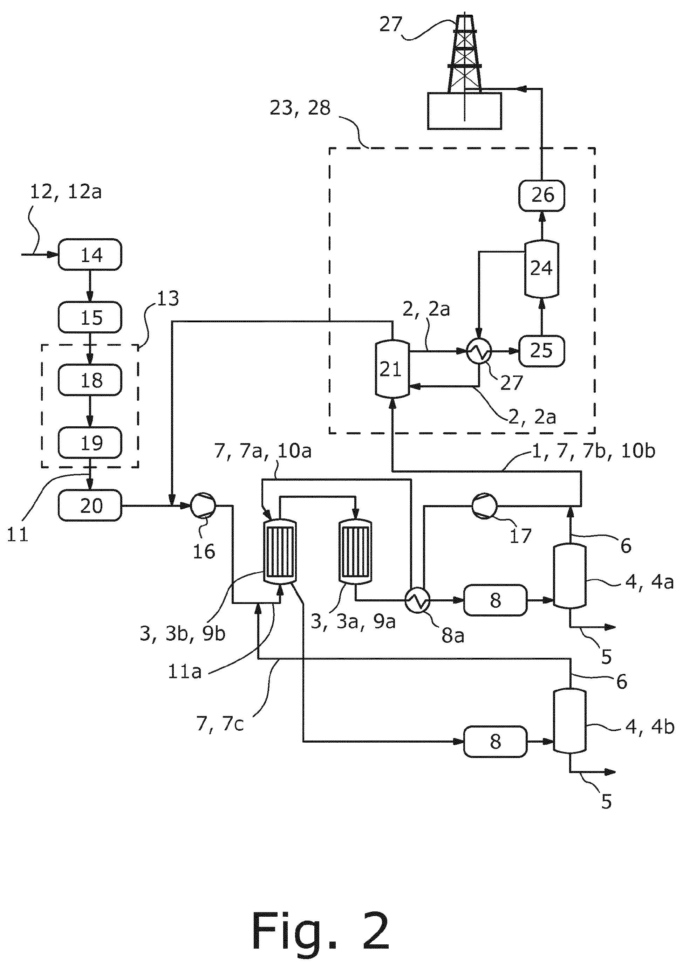

Bevorzugt ist, dass eine Abscheideanordnung 4 der Reaktoranordnung 3 zum Abscheiden eines Rohmethanolstroms 5 mit Wasser und Methanol aus dem Gasgemisch ein unreagiertes Restgas 6 mit Wasserstoff, Kohlenstoffmonoxid und Kohlenstoffdioxid abgibt und dass ein Recyclegasstrom 7 mit zumindest einem Teil des Restgases 6 der Reaktoranordnung 3 zur Umsetzung zugeführt wird. Bei dieser Umsetzung handelt es sich um die obige zumindest teilweise Umsetzung von Wasserstoff und Kohlenstoffoxiden in das Gasgemisch mit Wasser und Methanol. Die Abscheideanordnung 4 kann dabei - wie in dem ersten Ausführungsbeispiel gemäß der Fig. 1 - aus einem ersten Abscheider 4a oder - wie in dem zweiten Ausführungsbeispiel gemäß der Fig. 2 - aus einem ersten Abscheider 4a und einem zweiten Abscheider 4b bestehen, welche auch gemäß der Darstellung prozesstechnisch voneinander durch Teile der Reaktoranordnung 3 getrennt angeordnet sein können. Darauf wird untenstehend noch näher eingegangen. Jeweils dem ersten Abscheider 4a und ggf. auch dem zweiten Abscheider 4b prozesstechnisch vorgelagert kann eine Kühlvorrichtung 8 zum Kühlen des Gasgemischs vorgesehen sein, was in beiden dargestellten Ausführungsbeispielen der Fall ist. Aus dem Rohmethanolstrom 5 kann in an sich bekannter und hier nicht dargestellter Weise das Methanol gewonnen werden.

Wie oben festgestellt weist das erste Ausführungsbeispiel der Fig. 1 lediglich einen ersten Abscheider 4a auf. Ebenso gibt es nur einen ersten Rückstrom 7a, welcher als Recyclegasstrom 7 zu verstehen ist und welcher der Reaktoranordnung 3 zugeführt wird.

In dem zweiten Ausführungsbeispiel der Fig. 2 gibt es sowohl einen ersten Abscheider 4a als auch einen zweiten Abscheider 4b. Das zweite Ausführungsbeispiel der Fig. 2 weist insgesamt einen ersten Rückstrom 7a aus dem ersten Abscheider 4a, welcher dem adiabaten Reaktor 3b zugeführt wird, einen zweiten Rückstrom 7b, welcher ebenfalls aus dem ersten Abscheider 4a kommt aber dem isothermen Reaktor 3a zugeführt wird, und einen dritten Rückstrom 7c aus dem zweiten Abscheider 4b zur Zuführung ebenfalls zum isothermen Reaktor 3a auf. Der erste Rückstrom 7a, der zweite Rückstrom 7b und der dritte Rückstrom 7c können jeweils für sich als Recyclegasstrom 7 verstanden werden. Erkennbar ist, dass eine Zuführung des Recyclegasstroms 7 an prozesstechnisch unterschiedlichen Stellen der Reaktoranordnung 3 möglich ist. Das zweite Ausführungsbeispiel der Fig. 2 weist einen Wärmetauscher 8a auf, durch den jeweils der erste Rückstrom 7a und das Gasgemisch zum Wärmeaustausch geführt werden, sodass der erste Rückstrom 7a durch diesen Wärmeaustausch aufgewärmt wird und das Gasgemisch gekühlt wird.

Um das Auswaschen des Kohlenstoffdioxids an dem Recyclegasstrom 7 vorzunehmen ist es weiter bevorzugt, dass der Gasstrom 1 den Recyclegasstrom 7 zumindest teilweise umfasst, sodass das Kohlenstoffdioxid des Restgases zumindest teilweise durch das Waschmedium ausgewaschen wird. Dies entspricht dem zweiten Ausführungsbeispiel der Fig. 2, wobei hier speziell der Gasstrom 1 aus dem zweiten Rückstrom 7b besteht. Es wird also die Kohlenstoffdioxidwäsche an einem Strom von Gas durchgeführt, welcher aus der Reaktoranordnung 3 kommt. Alternativ oder zusätzlich könnte der Gasstrom 1 auch den dritten Rückstrom 7c zumindest teilweise umfassen oder bei Vorhandensein von weiteren derartigen Rückströmen einen dieser weiteren Rückströme zumindest teilweise umfassen.

Im Gegensatz hierzu erfolgt die Kohlenstoffdioxidwäsche im Ausführungsbeispiel der Fig. 1 prozesstechnisch der Reaktoranordnung 3 vorgelagert, was untenstehend noch genauer beschrieben wird. Gemäß einer hier nicht dargestellten Variante der Anlage der Fig. 1, welcher dieser bevorzugten Ausführungsform mit einem Auswaschen aus dem Recyclegasstrom 7 entsprechen würde, könnte der Gasstrom 1 zumindest einen Teil des ersten Rückstroms 7a umfassen.

Gemäß einer bevorzugten Ausführungsform ist vorgesehen, dass die Reaktoranordnung 3 eine Mehrzahl von prozesstechnisch seriell angeordneten Reaktorstufen 9a, b, jeweils zur katalytischen und zumindest teilweisen Umsetzung von Wasserstoff und Kohlenstoffoxiden in das Gasgemisch mit Wasser und Methanol, aufweist. Die vorliegenden Ausführungsbeispiele sehen eine erste solche Reaktorstufe 9a und eine zweite solche Reaktorstufe 9b vor. Es findet also eine entsprechende Methanolsynthese grundsätzlich sowohl in der ersten Reaktorstufe 9a als auch in der zweiten Reaktorstufe 9b statt, wobei sich die beiden Reaktorstufen 9a, b hinsichtlich ihrer Ausgestaltung sowie in den Reaktionsbedingungen unterscheiden können. Die erste Reaktorstufe 9a wird hier durch den adiabaten Reaktor 3b und die zweite Reaktorstufe 9b durch den isothermen Reaktor 3a gebildet. Es kann sein, dass die Abscheideanordnung 4 der prozesstechnisch letzten Reaktorstufe 9b der Reaktoranordnung 3 prozesstechnisch nachgelagert ist. Dieser Sachverhalt, welcher in dem ersten Ausführungsbeispiel der Fig. 1 verwirklicht ist, bedeutet, dass die Abscheideanordnung 4 das Gasgemisch von der letzten Reaktorstufe 9b empfängt. Er kann auch dadurch ausgedrückt werden, dass die Abscheideanordnung 4 der Reaktoranordnung 3 insgesamt mit der Mehrzahl der Reaktorstufen 9a, b prozesstechnisch nachgelagert ist.

In diesem Zusammenhang ist es grundsätzlich möglich, dass bei dem Vorhandensein einer solchen Vielzahl von Reaktorstufen 9a, b der Gasstrom 1 das Gasgemisch aus einer der Reaktorstufen 9a, b der Reaktoranordnung 3 ganz oder teilweise umfasst, sodass etwa bezogen auf die Reaktoranordnung 3 der Fig. 1 das Kohlenstoffdioxid durch das Waschmedium 2 aus demjenigen Gasgemisch ausgewaschen wird, welches aus der ersten Reaktorstufe 9a tritt. Diese Wäsche würde also vor der Zuführung des Gasgemischs in die zweite Reaktorstufe 9b erfolgen.

Hinsichtlich der obigen Abscheideanordnung 4 kann es aber auch sein - wie in dem zweiten Ausführungsbeispiel der Fig. 2 zu erkennen ist - dass die Abscheideanordnung 4 prozesstechnisch zwischen zwei Reaktorstufen 9a, b der Reaktoranordnung 3 angeordnet ist. Das bedeutet, dass die Abscheideanordnung 4 das Gasgemisch von einer Reaktorstufe erhält 9a, welche gemäß der prozesstechnisch seriellen Anordnung der Reaktorstufen 9a, b der prozesstechnisch letzten Reaktorstufe 9b vorgelagert ist.

Das unreagierte Restgas 6 aus der Abscheideanordnung 4 kann aufgeteilt werden. Daher ist es bevorzugt, dass ein erster Restgasstrom 10a des Restgases 6 einer prozesstechnisch der Abscheideanordnung 4 nachgelagerten Reaktorstufe 9b zur Umsetzung und ein zweiter Restgasstrom 10b des Restgases 6 einer der Abscheideanordnung 4 prozesstechnisch vorgelagerten Reaktorstufe 9a zur Umsetzung zugeführt wird. Das ist im Ausführungsbeispiel der Fig. 2 der Fall, in welchem der zweite Restgasstrom 10b der ersten Reaktorstufe 9a - also hier dem isothermen Reaktor 3a - und der erste Restgasstrom 10a der zweiten Reaktorstufe 9b, bei welchem es sich um den adiabaten Reaktor 3b handelt, zugeführt wird. Gemäß diesem Ausführungsbeispiel ist bevorzugt, dass der Gasstrom 1 - welcher vorschlagsgemäß der Wäsche unterzogen wird - den zweiten Restgasstrom 10b umfasst und insbesondere aus ihm besteht. Alternativ könnte der Gasstrom 1 auch den ersten Restgasstrom 10a umfassen oder aus ihm bestehen. Gemäß einer noch weiteren und hier nicht dargestellten Variante könnte auch das gesamte Restgas 6 vor Zuführung zur nachgelagerten Reaktorstufe 9b als Gasstrom 1 der vorschlagsgemäßen Wäsche zugeführt werden.

Eine geeignete Quelle für den Wasserstoff und die Kohlenstoffoxide kann durch die Reformierung eines kohlenstoffhaltigen Energieträgers wie etwa Erdgas oder dessen Bestandteile bereitgestellt werden. Entsprechend ist es - gemäß der Darstellung sowohl in der Fig. 1 als auch in der Fig. 2 bevorzugt, dass ein Frischgasstrom 11 mit Wasserstoff, Kohlenstoffmonoxid und Kohlenstoffdioxid zur Zuführung zu der Reaktoranordnung 3 durch eine Reformierung eines kohlenstoffhaltigen Energieträgerstroms 12 in einer Reformeranordnung 13 bereitgestellt wird. Bei dem Energieträgerstrom 12 kann es sich insbesondere um einen Erdgasstrom 12a handeln. Vor Zuführung des Energieträgerstroms 12 zu der Reformeranordnung 13 kann der Energieträgerstrom 12 einer Entschwefelungsvorrichtung 14 zur Entschwefelung des Energieträgerstroms 12 und anschließend einer Sättigungsvorrichtung 15 zur Sättigung des Energieträgerstroms 12 mit Wasserdampf zugeführt werden. Gemäß dem Ausführungsbeispiel der Fig. 1 kann es sich bei dem Frischgasstrom 11 um den Gasstrom 1 handeln, sodass das Kohlenstoffdioxid zumindest teilweise durch das Waschmedium 2 aus dem Frischgasstrom 11 ausgewaschen wird. Soweit der Frischgasstrom 11 prozesstechnisch auf weitere Art und Weise verarbeitet wird oder sich aus unterschiedlichen Quellen von Frischgas speist, kann es sich bei dem Gasstrom 1 um einen - vollständigen oder teilweisen - Strom von Frischgas an einer grundsätzlich beliebigen prozesstechnischen Stelle handeln.

Um den für die Methanolsynthese geeigneten Druck zu erreichen ist regelmäßig ein Synthesegaskompressor 16 vorgesehen, welcher das der Reaktoranordnung 3 zuzuführende Gas komprimiert. Soweit der Gasstrom 1 ebenfalls komprimiert wird, ist es bevorzugt, dass der Gasstrom 1 dem Synthesegaskompressor 16 zur Komprimierung des Gasstroms 1 dem Auswaschen prozesstechnisch nachgelagert zugeführt wird, und zwar vorzugsweise prozesstechnisch unmittelbar nachgelagert. Auf diese Weise kann die Menge an zu komprimierendem Gas reduziert werden. Dieser Sachverhalt lässt sich an beiden Ausführungsbeispielen erkennen. Hier ist es weiter bevorzugt, dass der Synthesegaskompressor 16 der Reaktoranordnung 3 prozesstechnisch unmittelbar vorgelagert ist.

Wenn auch ein Recyclegasstrom 7 wie oben beschrieben vorgesehen ist, wird regelmäßig der Frischgasstrom 11 mit dem Recyclegasstrom 7 zur Umsetzung in der Reaktoranordnung 3 zusammengeführt. Eine solche Zusammenführung kann auch vor der dann gemeinsamen Zuführung zur Reaktoranordnung 3 erfolgen. Im ersten Ausführungsbeispiel der Fig. 1 erfolgt die Zusammenführung des ersten Rückstroms 7a mit dem Frischgasstrom 11 zum Speisestrom 11a der Zuführung zu der Reaktoranordnung 3 prozesstechnisch vorgelagert, jedoch prozesstechnisch dem Synthesegaskompressor 16 nachgelagert Der Speisestrom 11a bezeichnet also hier und nachstehend die Gesamtheit des der Reaktoranordnung 3 zugeführten Gases.

Im zweiten Ausführungsbeispiel der Fig. 2 wird der zweite Rückstrom 7b dem Synthesegaskompressor 16 prozesstechnisch vorgelagert und der dritte Rückstrom 7c dem Synthesegaskompressor 16 prozesstechnisch nachgelagert mit dem Frischgasstrom 11 wiederum zum Speisestrom 11a zusammengeführt. Neben dem Synthesegaskompressor 16 ist auch jeweils ein Recyclekompressor 17 zur Komprimierung des Recyclegasstroms 7 vorgesehen, wobei er in beiden Ausführungsbeispielen speziell den ersten Rückstrom 7a komprimiert. Denkbar wären auch entsprechende Kompressoren zur Komprimierung des zweiten Rückstroms 7b und/oder des dritten Rückstroms 7c.

Gemäß der in der Fig. 1 dargestellten Variante wird also das Kohlenstoffdioxid aus dem Frischgasstrom 11 vor Zusammenführung mit dem Recyclegasstrom 7 zum Speisestrom 11a zumindest teilweise durch das Waschmedium ausgewaschen. Denkbar wäre aber auch, das Kohlenstoffdioxid aus dem Frischgasstrom 11 nach dieser Zusammenführung durch das Waschmedium zumindest teilweise auszuwaschen.

Eine bevorzugte Ausgestaltung der obigen Reformeranordnung 13 sieht vor, dass in einem Pre-Reformer 18 der Reformeranordnung 13 Kohlenwasserstoffe mit mindestens zwei Kohlenstoffatomen des Energieträgerstroms 12 in Methan, Wasserstoff und Kohlenstoffoxide aufgespalten werden. Ein solches Pre-Reforming ist an sich aus dem Stand der Technik als Reformierung bei niedriger Temperatur bekannt, wobei sich ebenfalls bekanntermaßen in dem Pre-Reformer 18 die Methanisierungsreaktion und die Wassergas-Shift-Reaktion im Wesentlichen im Gleichgewicht befinden. Als solche Kohlenwasserstoffe mit mindestens zwei Kohlenstoffatomen kommen insbesondere Ethan, Propan, Butan und Pentan infrage. Bevorzugt erfolgt das Aufspalten der Kohlenwasserstoffe in dem Pre-Reformer 18 in einer adiabaten Reaktion bei einer Temperatur von 400° C bis 500° C und vorzugsweise bei einem Druck von 25 bar bis 30 bar.

Diesem Pre-Reformer 18 kann eine weitere Reformierungsstufe prozesstechnisch nachgelagert sein, in welcher auch das Methan in einer Reformierungsreaktion umgewandelt wird. So ist es bevorzugt, dass in einem Reformer 19 der Reformeranordnung 13 Methan des Energieträgerstroms 12 in Wasserstoff und Kohlenstoffoxide aufgespalten wird. Dieses Aufspalten erfolgt insbesondere durch die aus dem Stand der Technik und an sich bekannte Reformierung von Methan - sowie ggf. vorhandenen höheren Kohlenwasserstoffen - unter Anwesenheit von Wasser in Wasserstoff und Kohlenstoffmonoxid. Bevorzugt erfolgt das Aufspalten des Methans in dem Reformer 19 durch eine autotherme Reformierung mit Sauerstoff, sodass eine katalytische partielle Oxidation die für die endothermen Reformierungsreaktionen erforderliche Wärme bereitstellt. Endotherm ist insbesondere die oben genannte Reformierung von Methan. Bevorzugt ist weiter, dass der Reformer 19 eine Austrittstemperatur von 800° C bis 1000° C aufweist. Wie in den Ausführungsbeispielen der Fig. 1 und der Fig. 2 dargestellt, kann der Reformeranordnung 13 prozesstechnisch nachgelagert eine Abhitzeanordnung 20 zum Abkühlen des Frischgasstroms 11 -welcher wie oben beschrieben aus der Reformeranordnung 13 erhalten wird - vorgesehen sein. Auf diese Weise kann in dem Frischgasstrom 11 verbliebenes Wasser zumindest teilweise auskondensiert werden.

Nun wird auf die Einzelheiten der vorschlagsgemäßen Kohlenstoffdioxid-Wäsche im engeren Sinne eingegangen. Das Waschmedium 2 kann gemäß einer Variante aus einer wässrigen Lösung 2a von Ammoniak und Kohlenstoffdioxid bestehen. Grundsätzlich ist es bevorzugt, dass das molare Verhältnis von Ammoniak zu Kohlenstoffdioxid in der Lösung 2a zwischen 1 und 4 beträgt. Dabei ist es besonders günstig, wenn das molare Verhältnis von Ammoniak zu Kohlenstoffdioxid in der Lösung 2a zwischen 1,5 und 3 beträgt.

Eine bevorzugte Ausführungsform des vorschlagsgemäßen Verfahrens ist dadurch gekennzeichnet, dass der Gasstrom 1 zumindest teilweise einer Absorptionsstufe 21 zur Aufnahme des Kohlenstoffdioxids in das Waschmedium 2 zugeführt wird. Hierzu wird in dem Ausführungsbeispiel der Fig. 1 gemäß einer bevorzugten Variante aus dem Gasstrom 1 ein Teilstrom 22 abgezweigt, welcher Teilstrom 22 der Absorptionsstufe 14 zugeführt wird. Anschließend wird dieser Teilstrom 22 zum verbliebenen Gasstrom 1 zurückgeführt. Alternativ und wie für das Ausführungsbeispiel der Fig. 2 dargestellt, kann auch der gesamte Gasstrom 1 der Absorptionsstufe 21 zugeführt werden.

In der Absorptionsstufe 21 nimmt insbesondere die Lösung 2a das Kohlenstoffdioxid aus dem Gasstrom 1 auf, was regelmäßig durch Reaktion mit dem im Wasser aufgelösten Ammoniak zu der ggf. zusätzlichen Bildung von Ammoniumhydrogencarbonat (NH₄HCO₃) in der Lösung 2a führt. Dabei ist der Reinigungsgrad, also speziell der Stoffanteil des von dem Waschmedium 2 aufgenommenen Kohlenstoffdioxids aus dem Gasstrom 1 von verschiedenen Parametern wie Druck, Temperatur etc. abhängig und insoweit auch einstellbar. Vorzugsweise wird dabei das Kohlenstoffdioxid zu einem molaren Anteil von mindestens 95 % aus dem Teilstrom 22 bzw. aus dem Gasstrom 1 durch das Waschmedium 2 ausgewaschen. Dieser molare Anteil kann auch mindestens 99 % und insbesondere mindestens 99,9 % betragen.

Grundsätzlich kann es in der Absorptionsstufe 21 dann auch zu einer Sättigung der Lösung 2a mit Kohlenstoffdioxid kommen. Wie in der Fig. 1 dargestellt ist es bevorzugt, dass das Waschmedium 2 in einer Waschanordnung 23, welche die bereits beschriebene Absorptionsstufe 21 und eine Regenerationsstufe 24 zur Abgabe von Kohlenstoffdioxid aus dem Waschmedium 2 aufweist, in einem Kreislauf gefahren wird. In an sich bekannter Weise gibt also das Waschmedium 2 in der Regenerationsstufe 24 im Wesentlichen das in der Absorptionsstufe 21 aufgenommene Kohlenstoffdioxid wieder ab.

Eine erste denkbare Möglichkeit zur Abgabe des Kohlenstoffdioxids in der Regenerationsstufe 24 besteht darin, das auf diese Weise mit Kohlenstoffdioxid angereicherte Waschmedium 2 sich ausdehnen zu lassen. Bevorzugt ist jedoch, dass das Kohlenstoffdioxid aus dem Waschmedium 2 in der Regenerationsstufe 24 bei im Wesentlichen gleichbleibendem Volumen des Waschmediums 2 - also ohne wesentliche Ausdehnung - abgegeben wird. Bezüglich des Druckes ist bevorzugt, dass das Kohlenstoffdioxid aus dem Waschmedium 2 in der Regenerationsstufe 24 bei zumindest gleichbleibendem Druck des Waschmediums 2 abgegeben wird. Nach dieser Variante ist also gefordert, dass der Druck des Waschmediums 2 bei der Abgabe in der Regenerationsstufe 24 jedenfalls nicht sinkt.

Eine bevorzugte Variante für eine solche Abgabe ist dadurch gekennzeichnet, dass die Waschanordnung 23 eine Heizvorrichtung 25 zum Erhitzen des Waschmediums 2 aufweist, sodass das Waschmedium 2 in der Regenerationsstufe 24 eine höhere Temperatur als in der Absorptionsstufe 21 aufweist. Dieses Erhitzen kann in einer bevorzugten Variante dazu führen, dass das Kohlenstoffdioxid in der Regenerationsstufe 24 auch mit einem höheren Partialdruck abgegeben wird, als das Kohlenstoffdioxid des Gasstroms 1 in der Absorptionsstufe 21 aufweist. Vorzugsweise ist in der Waschanordnung 23 noch eine Nachwäsche 26 zur Reinigung des abgegebenen Kohlenstoffdioxids von Ammoniak vorgesehen. Ebenso vorteilhaft ist das Vorsehen eines Wäscher-Wärmetauschers 27 zum gegenläufigen Wärmeaustausch des im Kreislauf gefahrenen Waschmediums 2. Auf diese Weise kann eine Abkühlung des von der Regenerationsstufe 24 zur Absorptionsstufe 21 gefahrenen Waschmediums 2 bei gleichzeitiger Erwärmung des von der Absorptionsstufe 21 zur Regenerationsstufe 24 gefahrenen Waschmediums 2 erreicht werden.

Der durch das vorschlagsgemäße Verfahren höhere erreichbare Druck bei der Abgabe des Kohlenstoffdioxids ist besonders dann vorteilhaft wenn, wie für beide Ausführungsbeispiele dargestellt, das in der Regenerationsstufe 24 abgegebene Kohlenstoffdioxid zur Speicherung in eine unterirdische Lagerstätte 27 injiziert wird. Dieses Injizieren erfolgt vorzugsweise in an sich bekannter Weise durch ein Verpressen in die unterirdische Lagerstätte 27. Alternativ kann das in der Regenerationsstufe 24 abgegebene Kohlenstoffdioxid auch als Purgegas in die Atmosphäre abgegeben oder einer anderen Weiterverarbeitung zugeführt werden.

Bezüglich des Drucks des Gasstroms 1 bei der Wäsche ist es bevorzugt, dass das Kohlenstoffdioxid bei einem Druck des Gasstroms 1 von mindestens 20 bar, vorzugsweise von mindestens 40 bar, insbesondere von mindestens 50 bar, ausgewaschen wird. Durch diesen hohen Druck des Gasstroms 1 auf der Seite der Absorptionsstufe 21 wird auch das Erreichen eines entsprechend hohen Drucks des Kohlenstoffdioxids auf der Seite der Regenerationsstufe 24 erleichtert.

Schließlich ist es bevorzugt, dass das Kohlenstoffdioxid bei einer Temperatur des Gasstroms 1 von mindestens 30° C, vorzugsweise von mindestens 40° C und insbesondere von mindestens 45° C ausgewaschen wird.

Die vorschlagsgemäße Anlage zur Synthese von Methanol weist einen Wäscher 28 zum zumindest teilweisen Auswaschen von Kohlenstoffdioxid durch ein Waschmedium 2 aus einem Gasstrom 1 umfassend Wasserstoff und Kohlenstoffdioxid auf. Ein solcher Wäscher 28 kann die obige Waschanordnung 23 umfassen oder - wie in der Fig. 1 und der Fig. 2 gezeigt - aus dieser bestehen.

Vorschlagsgemäß weist diese Anlage ebenso eine Reaktoranordnung 3 auf, welcher der Gasstrom 1 prozesstechnisch nach dem Wäscher 28 zur katalytischen und zumindest teilweisen Umsetzung von Wasserstoff und Kohlenstoffoxiden in ein Gasgemisch mit Wasser und Methanol zugeführt wird. Die vorschlagsgemäße Anlage ist dadurch gekennzeichnet, dass das Waschmedium 2 eine wässrige Lösung 2a von Ammoniak zum Auswaschen des Kohlenstoffdioxids umfasst.

Weitere bevorzugte Ausführungsformen und Merkmale der vorschlagsgemäßen Anlage ergeben sich aus den bevorzugten Ausführungsformen und Merkmalen des vorschlagsgemäßen Verfahrens.

## Patentansprüche

1. Verfahren zur Synthese von Methanol, wobei aus einem Gasstrom (1) mit Wasserstoff und Kohlenstoffdioxid das Kohlenstoffdioxid zumindest teilweise durch ein Waschmedium (2) ausgewaschen wird und wobei nach dem Auswaschen der Gasstrom (1) einer Reaktoranordnung (3) zur katalytischen und zumindest teilweisen Umsetzung von Wasserstoff und Kohlenstoffoxiden in ein Gasgemisch mit Wasser und Methanol zugeführt wird,
**dadurch gekennzeichnet,**
**dass** das Waschmedium (2) eine wässrige Lösung (2a) von Ammoniak zum Auswaschen des Kohlenstoffdioxids umfasst.

2. Verfahren nach Anspruch 1, **dadurch gekennzeichnet, dass** eine Abscheideanordnung (4) der Reaktoranordnung (3) zum Abscheiden eines Rohmethanolstroms (5) mit Wasser und Methanol aus dem Gasgemisch ein unreagiertes Restgas (6) mit Wasserstoff, Kohlenstoffmonoxid und Kohlenstoffdioxid abgibt und dass ein Recyclegasstrom (7) mit zumindest einem Teil des Restgases (6) der Reaktoranordnung (3) zur Umsetzung zugeführt wird.

3. Verfahren nach Anspruch 2, **dadurch gekennzeichnet, dass** der Gasstrom (1) den Recyclegasstrom (7) zumindest teilweise umfasst, sodass das Kohlenstoffdioxid des Restgases (6) zumindest teilweise durch das Waschmedium (2) ausgewaschen wird.

4. Verfahren nach einem der Ansprüche 1 bis 3, **dadurch gekennzeichnet, dass** die Reaktoranordnung (3) eine Mehrzahl von prozesstechnisch seriell angeordneten Reaktorstufen (9a, b), jeweils zur katalytischen und zumindest teilweisen Umsetzung von Wasserstoff und Kohlenstoffoxiden in das Gasgemisch mit Wasser und Methanol, aufweist, vorzugsweise, dass die Abscheideanordnung (4) der prozesstechnisch letzten Reaktorstufe (9b) der Reaktoranordnung (3) prozesstechnisch nachgelagert ist.

5. Verfahren nach Anspruch 4, **dadurch gekennzeichnet, dass** die Abscheideanordnung (4) prozesstechnisch zwischen zwei Reaktorstufen (9a, b) der Reaktoranordnung (3) angeordnet ist, vorzugsweise, dass ein erster Restgasstrom (10a) des Restgases (6) einer prozesstechnisch der Abscheideanordnung (4) nachgelagerten Reaktorstufe (9b) zur Umsetzung und ein zweiter Restgasstrom (10b) des Restgases (6) einer der Abscheideanordnung (4) prozesstechnisch vorgelagerten Reaktorstufe (9a) zur Umsetzung zugeführt wird, insbesondere, dass der Gasstrom (1) den ersten Restgasstrom (10a) oder den zweiten Restgasstrom (10b) umfasst.

6. Verfahren nach einem der Ansprüche 1 bis 5, **dadurch gekennzeichnet, dass** der Gasstrom (1) einem Synthesegaskompressor (16) zur Komprimierung des Gasstroms (1) dem Auswaschen prozesstechnisch vorzugsweise unmittelbar nachgelagert zugeführt wird, insbesondere, dass der Synthesegaskompressor (16) der Reaktoranordnung (3) prozesstechnisch unmittelbar vorgelagert ist.

7. Verfahren nach einem der Ansprüche 1 bis 6, **dadurch gekennzeichnet, dass** ein Frischgasstrom (11) mit Wasserstoff, Kohlenstoffmonoxid und Kohlenstoffdioxid zur Zuführung zu der Reaktoranordnung (3) durch eine Reformierung eines kohlenstoffhaltigen Energieträgerstroms (12) in einer Reformeranordnung (13) bereitgestellt wird, vorzugsweise, dass der Frischgasstrom (11) der Gasstrom (1) ist, insbesondere, dass der Frischgasstrom (11) mit dem Recyclegasstrom (7) zur Umsetzung in der Reaktoranordnung (3) zusammengeführt wird, weiter insbesondere, dass das Kohlenstoffdioxid aus dem Frischgasstrom (11) vor Zusammenführung mit dem Recyclegasstrom (7) zumindest teilweise durch das Waschmedium ausgewaschen wird.

8. Verfahren nach einem der Anspruch 7, **dadurch gekennzeichnet, dass** in einem Pre-Reformer (18) der Reformeranordnung (13) Kohlenwasserstoffe mit mindestens zwei Kohlenstoffatomen des Energieträgerstroms (12) in Methan, Wasserstoff und Kohlenstoffoxide aufgespalten werden, insbesondere, dass das Aufspalten der Kohlenwasserstoffe in dem Pre-Reformer (18) in einer adiabaten Reaktion bei einer Temperatur von 400° C bis 500° C erfolgt, vorzugsweise, bei einem Druck von 25 bar bis 30 bar erfolgt.

9. Verfahren nach Anspruch 7 oder 8, **dadurch gekennzeichnet, dass** in einem Reformer (19) der Reformeranordnung (13) Methan des Energieträgerstroms (12) in Wasserstoff und Kohlenstoffoxide aufgespalten wird, insbesondere, dass das Aufspalten des Methans in dem Reformer (19) durch eine autotherme Reformierung mit Sauerstoff erfolgt, sodass eine katalytische partielle Oxidation die für die endothermen Reformierungsreaktionen erforderliche Wärme bereitstellt, vorzugsweise, dass der Reformer (19) eine Austrittstemperatur von 800° C bis 1000° C aufweist.

10. Verfahren nach einem der Ansprüche 1 bis 9, **dadurch gekennzeichnet, dass** das Waschmedium (2) im Wesentlichen aus einer wässrigen Lösung (2a) von Ammoniak und Kohlenstoffdioxid besteht, vorzugsweise, dass das molare Verhältnis von Ammoniak zu Kohlenstoffdioxid in der Lösung (2a) zwischen 1 und 4 beträgt, insbesondere, dass das molare Verhältnis von Ammoniak zu Kohlenstoffdioxid in der Lösung (2a) zwischen 1,5 und 3 beträgt.

11. Verfahren nach einem der Ansprüche 1 bis 10, **dadurch gekennzeichnet, dass** der Gasstrom (1) zumindest teilweise einer Absorptionsstufe (21) zur Aufnahme des Kohlenstoffdioxids in das Waschmedium (2) zugeführt wird, vorzugsweise, dass das Waschmedium (2) in einer Waschanordnung (23), welche die Absorptionsstufe (21) und eine Regenerationsstufe (24) zur Abgabe von Kohlenstoffdioxid aus dem Waschmedium (2) aufweist, in einem Kreislauf gefahren wird.

12. Verfahren nach Anspruch 11, **dadurch gekennzeichnet, dass** das Kohlenstoffdioxid aus dem Waschmedium (2) in der Regenerationsstufe (24) bei im Wesentlichen gleichbleibendem Volumen, insbesondere bei zumindest gleichbleibendem Druck, des Waschmediums (2) abgegeben wird.

13. Verfahren nach Anspruch 11 oder 12, **dadurch gekennzeichnet, dass** die Waschanordnung (23) eine Heizvorrichtung (25) zum Erhitzen des Waschmediums (2) aufweist, sodass das Waschmedium (2) in der Regenerationsstufe (24) eine höhere Temperatur als in der Absorptionsstufe (21) aufweist, vorzugsweise, sodass das Kohlenstoffdioxid in der Regenerationsstufe (24) mit einem höheren Partialdruck abgegeben wird, als das Kohlenstoffdioxid des Gasstroms (1) in der Absorptionsstufe (21) aufweist.

14. Verfahren nach einem der Ansprüche 11 bis 13, **dadurch gekennzeichnet, dass** das in der Regenerationsstufe (24) abgegebene Kohlenstoffdioxid zur Speicherung in eine unterirdische Lagerstätte (27) injiziert wird.

15. Anlage zur Synthese von Methanol mit einem Wäscher (28) zum zumindest teilweisen Auswaschen von Kohlenstoffdioxid durch ein Waschmedium (2) aus einem Gasstrom (1) umfassend Wasserstoff und Kohlenstoffdioxid und mit einer Reaktoranordnung (3), welcher der Gasstrom (1) prozesstechnisch nach dem Wäscher (28) zur katalytischen und zumindest teilweisen Umsetzung von Wasserstoff und Kohlenstoffoxiden in ein Gasgemisch mit Wasser und Methanol zugeführt wird,
**dadurch gekennzeichnet,**
**dass** das Waschmedium (2) eine wässrige Lösung (2a) von Ammoniak zum Auswaschen des Kohlenstoffdioxids umfasst.
